(19) Europäisches Patentamt
European Patent Office
Office européen des brevets

(11) Veröffentlichungsnummer: **0 238 443 B1**

(12) **EUROPÄISCHE PATENTSCHRIFT**

(45) Veröffentlichungstag der Patentschrift: **10.03.93**

(51) Int. Cl.$^5$: **C09B 3/04**, D06P 3/52, C08K 5/16, C07C 255/00

(21) Anmeldenummer: **87810142.7**

(22) Anmeldetag: **12.03.87**

# (54) Dicyanobenzanthronverbindungen.

(30) Priorität: **18.03.86 CH 1088/86**

(43) Veröffentlichungstag der Anmeldung:
**23.09.87 Patentblatt 87/39**

(45) Bekanntmachung des Hinweises auf die Patenterteilung:
**10.03.93 Patentblatt 93/10**

(84) Benannte Vertragsstaaten:
**CH DE FR GB IT LI**

(56) Entgegenhaltungen:
**US-A- 1 628 280**
**US-A- 2 117 720**

(73) Patentinhaber: **CIBA-GEIGY AG**
**Klybeckstrasse 141**
**CH-4002 Basel(CH)**

(72) Erfinder: **Schwander, Hansrudolf, Dr.**
**Unterm Schellenberg 189**
**CH-4125 Riehen(CH)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 238 443 B1

**Beschreibung**

Die vorliegende Erfindung betrifft neue Dicyanobenzanthronverbindungen, Verfahren zu deren Herstellung sowie deren Verwendung als Farbstoffe zum Färben und Bedrucken von halbsynthetischem oder synthetischem hydrophobem Material.

Die neuen Dicyanobenzanthronverbindungen entsprechen der Formel

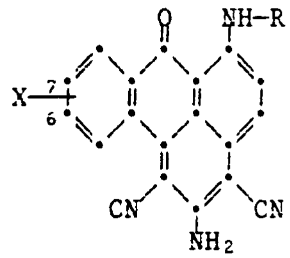

(1)

worin

R Wasserstoff, eine gegebenenfalls substituierte Alkyl- oder Arylgruppe und

X Wasserstoff oder Halogen, das sich in 6- oder 7-Stellung befindet, bedeutet.

Bedeutet R einen Alkylrest, so handelt es sich um einen gegebenenfalls substituierten unverzweigten oder verzweigten Alkylrest oder um einen Cycloalkylrest. Der Cycloalkylrest weist vorzugsweise 5 bis 8 C-Atome, der offenkettige Alkylrest vorzugsweise 1 bis 8 C-Atome auf.

Als unverzweigte oder verzweigte offenkettige Alkylreste kommen z.B. in Frage: Methyl, Ethyl, n- und iso-Propyl, n-, sec.-oder tert.Butyl, n- und iso-Pentyl, n- und iso-Hexyl oder 2-Ethylhexyl.

Diese Alkylreste können ein- oder mehrmals substituiert sein, beispielsweise durch OH, $C_1$-$C_4$-Alkoxy, durch OH substituiertes $C_1$-$C_4$-Alkoxy, Phenyl, Phenoxy, oder Phenylaminocarbonyl, wobei die Phenylgruppe in den drei letztgenannten Resten z.B. durch $C_1$-$C_4$-Alkyl, $C_1$-$C_4$-Alkoxy, Sulfo oder Phenoxy substituiert sein kann. Geeignete Reste dieser Art sind z.B.: Hydroxyethyl, 1-Hydroxy-isopropyl, Ethoxymethyl, 2-Hydroxyethoxypentyl, Benzyl, 1-Phenylethyl, 2-Phenylethyl, 1-Methyl-2-phenylethyl, 1-iso-Butyl-3-phenylpropyl, 1,5-Diphenylpentyl-3, 1-Methyl-2-phenoxyethyl oder 1-Methyl-2-phenylaminocarbonyl-ethyl.

Bedeutet R einen gegebenenfalls substituierten $C_5$-$C_8$-Cycloalkylrest, so handelt es sich vor allem um den Cyclopentyl- und Cyclohexylrest; als Substituenten kommen vor allem $C_1$-$C_4$-Alkylgruppen, insbesondere die $CH_3$-Gruppe, in Frage.

Bedeutet R einen Arylrest, so handelt es sich vor allem um einen Naphthylrest und insbesondere um einen Phenylrest, wobei diese Reste substituiert sein können, z.B. durch $C_1$-$C_4$-Alkyl, $C_1$-$C_4$-Alkoxy, Sulfo, Halogen, wie Fluor, Chlor oder Brom, Nitro, $C_1$-$C_4$-Alkylcarbonylamino oder $C_1$-$C_4$-Alkoxycarbonyl.

In bevorzugten Farbstoffen der Formel (1) bedeutet

R $C_1$-$C_6$-Alkyl, welches unsubstituiert ist oder durch Phenyl, Phenoxy oder Phenylaminocarbonyl substituiert ist, wobei die Phenylgruppe in den drei letztgenannten Resten durch $C_1$-$C_4$-Alkyl, $C_1$-$C_4$-Alkoxy oder Phenoxy substituiert sein kann, oder Phenyl, welches unsubstituiert ist oder durch $C_1$-$C_4$-Alkyl substituiert ist.

In den Farbstoffen der Formel (1) bedeutet X Halogen, wie Brom oder vor allem Chlor, welches sich in 6- oder 7-Stellung befindet, oder insbesondere Wasserstoff.

Die neuen Dicyanobenzanthronverbindungen der Formel (1) erhält man z.B., indem man ein Aminoanthrachinon der Formel

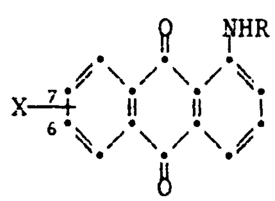

(2)

worin X und R die unter der Formel (1) angegebene Bedeutung aufweisen, in einem inerten Lösungsmittel in Gegenwart von Titantetrachlorid und einem tert.Amin mit Malodinitril umsetzt.

Als inerte Lösungsmittel kommen z.B. aliphatische Kohlenwasserstoffe, wie n-Pentan, n-Hexan oder n-Heptan, chlorierte Kohlenwasserstoffe, wie Methylenchlorid, Chloroform oder Tetrachlormethan, Ether, wie,

z.B. Diethylether, oder aromatische Verbindungen, wie z.B. Nitrobenzol oder Halogenbenzole in Betracht. Vorzugsweise verwendet man einen chlorierten Kohlenwasserstoff, vor allem Methylenchlorid.

Als tert.Amin werden z.B. aliphatische Amine, wie Triethylamin oder vor allem aromatische Amine, wie z.B. Picolin oder Pyridin verwendet.

Pro Mol Anthrachinonverbindung der Formel (2) setzt man mindestens 2 Mol, vorzugsweise jedoch 2,2 bis 3 Mol Malodinitril ein. Ein grösserer Ueberschuss ist im allgemeinen nicht schädlich, jedoch unzweckmässig.

Die Menge an Titantetrachlorid beträgt vorzugsweise 2 bis 10 Mol, insbesondere 3 bis 6 Mol pro Mol Anthrachinonverbindung der Formel (2).

Das tertiäre Amin wird mindestens in der Menge eingesetzt, die erforderlich ist, um die entstehende Säure zu binden. Im allgemeinen verwendet man jedoch einen Ueberschuss, vorzugsweise 2 bis 6 Mol pro Mol Titantetrachlorid.

Die Reaktionstemperatur liegt im allgemeinen etwa zwischen -10° und +60°C, vorzugsweise zwischen 0° und 25°C.

Es muss als überraschend bezeichnet werden, dass bei der Umsetzung der Anthrachinonverbindung der Formel (2) mit Malodinitril eine Dicyanobenzanthronverbindung entsteht.

Die erfindungsgemässen Verbindungen der Formel (1) können als Farbstoffe zum Färben und Bedrukken von halbsynthetischen und, insbesondere, synthetischen hydrophoben Fasermaterialien, vor allem Textilmaterialien, verwendet werden. Textilmaterialien aus Mischgeweben, die derartige halbsynthetische bzw. synthetische hydrophobe Textilmaterialien enthalten, können ebenfalls mit Hilfe der erfindungsgemässen Verbindungen gefärbt oder bedruckt werden.

Als halbsynthetische Textilmaterialien kommen vor allem Cellulose-2½-Acetat und Cellulosetriacetat in Frage.

Synthetische hydrophobe Textilmaterialien bestehen vor allem aus linearen, aromatischen Polyestern, beispielsweise solchen aus Terephthalsäure und Glykolen, besonders Ethylenglykol oder Kondensationsprodukten aus Terephthalsäure und 1,4-Bis-(hydroxymethyl)-hexahydrobenzol; aus Polycarbonaten, z.B. solchen aus α, α-Dimethyl-4,4′-dihydroxy-diphenylmethan und Phosgen, aus Fasern auf Polyvinylchlorid- sowie Polyamid-Basis.

Die Applikation der erfindungsgemässen Verbindungen auf die Textilmaterialien erfolgt nach bekannten Färbeverfahren. Beispielsweise färbt man Polyesterfasermaterialien im Ausziehverfahren aus wässriger Dispersion in Gegenwart von üblichen anionischen oder nichtionischen Dispergiermitteln und gegebenenfalls üblichen Quellmitteln (Carrier) bei Temperaturen zwischen 80 und 140°C. Cellulose-2½-acetat färbt man vorzugsweise zwischen ungefähr 65 bis 85°C und Cellulosetriacetat bei Temperaturen bis zu 115°C.

Die neuen Farbstoffe färben im Färbebad gleichzeitig anwesende Wolle und Baumwolle nicht oder nur wenig an (sehr gute Reserve), so dass sie auch gut zum Färben des Polyesteranteils in Polyester/Wolle-und Polyester/Cellulosefaser-Mischungen vewendet werden können.

Die erfindungsgemässen Farbstoffe eignen sich besonders zum Färben nach dem Thermosol-Verfahren und für den Textildruck.

Das genannte Textilmaterial kann in den verschiedensten Verarbeitungsformen vorliegen, wie z.B. als Faser, Faden oder Vlies, als Gewebe oder Gewirke.

Es ist vorteilhaft, die erfindungsgemässen Farbstoffe vor ihrer Verwendung in ein Farbstoffpräparat überzuführen. Hierzu wird der Farbstoff vermahlen, so dass seine Teilchengrösse im Mittel zwischen 0,01 und 10 Mikrometer beträgt. Das Vermahlen kann in Gegenwart von Dispergiermitteln erfolgen. Beispielsweise wird der getrocknete Farbstoff mit einem Dispergiermittel gemahlen oder in Pastenform mit einem Dispergiermittel geknetet und hierauf im Vakuum oder durch Zerstäuben getrocknet. Mit den so erhaltenen Präparaten kann man nach Zugabe von Wasser färben und bedrucken.

Beim Bedrucken wird man die üblichen Verdickungsmittel verwenden, z.B. modifizierte oder nichtmodifizierte natürliche Produkte, beispielsweise Alginate, Britishgummi, Gummi arabicum, Kristallgummi, Johannisbrotkernmehl, Tragant, Carboxymethylcellulose, Hydroxyethylcellulose, Stärke oder synthetische Produkte, beispielsweise Polyacrylamide, Polyacrylsäure oder deren Copolymere oder Polyvinylalkohole.

Die erfindungsgemässen Farbstoffe sind praktisch unempfindlich gegen Carrier und verleihen den genannten Materialien, vor allem dem Polyestermaterial egale blaustichig rot bis violette Farbtöne von sehr guten Gebrauchs-Echtheiten, wie vor allem guter Lichtechtheit, guter Sublimierechtheit, Thermofixier-, Plissier-, Chlor- und Nassechtheit wie Wasser-, Schweiss- und Waschechtheit; die Ausfärbungen sind ferner gekennzeichnet durch eine gute pH-Stabilität und sehr gute Reibechtheit. Es werden ausserdem sehr farbstarke Färbungen erzielt, die kein "catalytic fading" zeigen.

Die erfindungsgemässen Farbstoffe können auch gut verwendet werden zur Herstellung von Mischnuancen zusammen mit anderen Farbstoffen. Selbstverständlich können auch Mischungen der erfindungsgemässen Farbstoffe untereinander verwendet werden.

Die neuen Farbstoffe eignen sich auch zum Färben und Pigmentieren von hochmolekularen organischen Materialien, z.B. von Celluloseäthern und -estern, wie Aethylcellulose, Nitrocellulose, Celluloseacetat, Cellulosebutyrat, natürlichen Harzen oder Kunstharzen, wie Polymerisationsharzen oder Kondensationsharzen, z.B. Aminoplasten, insbesondere Harnstoff- und Melamin-Formaldehydharzen, Alkydharzen, Phenoplasten, Polycarbonaten, Polyolefinen, wie Polystyrol, Polyvinylchlorid, Polyethylen, Polypropylen, Polyacrylnitril, Polyacrylsäureester, Polyamiden, Polyurethanen oder Polyestern, Gummi, Casein, Silikon und Silikonharzen, einzeln oder in Mischungen.

Dabei spielt es keine Rolle, ob die erwähnten hochmolekularen Verbindungen als plastische Massen, Schmelzen oder in Form von Spinnlösungen, Lacken, Anstrichstoffen oder Druckfarben vorliegen. Je nach Verwendungszweck erweist es sich als vorteilhaft, die neuen Farbstoffe als Toner oder in Form von Präparaten zu verwenden.

Die Farbstoffe können in der Form, wie sie in der Synthese anfallen, eingesetzt werden. In leicht gemahlener Form liefern sie opake Ausfärbungen. Man kann sie aber auch einer intensiveren Mahlung unterziehen und erhält dann transparente Ausfärbungen, wie beispielsweise farbstarke Metalleffektlackierung.

Thermoplastische Kunststoffe, die mit den neuen Farbstoffen der Formel (1) in der Schmelze gefärbt werden können, sind Polystyrol und deren Mischpolymerisate, Polycarbonate, Polyamide, insbesondere aber lineare Polyester.

Als lineare Polyester seien insbesondere jene erwähnt, die durch Polykondensation von Terephthalsäure oder deren Estern mit Glykolen der Formel $HO-(CH_2)_n-OH$, worin n die Zahl 2-10 bedeutet, oder mit 1,4-Di-(hydroxymethyl)-cyclohexan oder durch Polykondensation von Glykolethern von Hydroxybenzoesäuren, beispielsweise p-($\beta$-Hydroxyethoxy)-benzoesäure erhalten werden. Der Begriff lineare Polyester umfasst auch Copolyester, die durch teilweisen Ersatz der Terephthalsäure durch eine andere Dicarbonsäure oder eine Hydroxycarbonsäure und/oder durch teilweisen Ersatz des Glykols durch ein anderes Diol erhalten werden.

Von besonderem Interesse sind jedoch die Polyethylen-terephthalate.

Die zu färbenden linearen Polyester zweckmässig in Form von Pulver, Schnitzeln oder Granulaten mit dem Farbstoff innig vermischt. Dies kann beispielsweise durch Panieren der Polyesterteilchen mit dem fein verteilten trockenen Farbstoffpulver oder durch Behandeln der Polyesterteilchen mit einer Lösung oder Suspension des Farbstoffes in einem organischen Lösungsmittel und nachherige Entfernung des Lösungsmittels geschehen.

Schliesslich kann die zu färbende Substanz auch direkt dem geschmolzenen Polyester oder auch vor oder während der Polykondensation des Polyethylenterephthalates zugegeben werden.

Das Verhältnis von Farbstoff zu Polyester kann, je nach der gewünschten Farbstärke, innerhalb weiter Grenzen schwanken. Im allgemeinen empfiehlt sich die Verwendung von 0,01-2 Teilen Farbstoff auf 100 Teile Polyester.

Die so behandelten Polyesterteilchen werden nach bekannten Verfahren im Extruder geschmolzen und zu Gegenständen, insbesondere Folien oder Fasern, ausgepresst oder zu Platten gegossen.

Man erhält gleichmässig und intensiv blaustichig rot bis violett gefärbte Gegenstände von hoher Licht- oder Migrationsechtheit. Die verfahrensgemäss erhältlichen gefärbten Fasern zeichnen sich ausserdem durch hervorragende Nass- und Trockenreinigungsechtheiten aus.

Ein besonderer Vorteil der erfindungsgemäss zu verwendenden Farbstoffe besteht darin, dass sie sich in der Polyesterschmelze lösen und hohe Temperaturen bis 300 °C aushalten, ohne sich zu zersetzen, so dass bedeutend klarere Färbungen erhalten werden als bei Verwendung unlöslicher Pigmente.

Die vorstehend genannte Verwendung der erfindungsgemässen Dicyanobenzanthronverbindungen der Formel (1) stellt ebenso einen Gegenstand der vorliegenden Erfindung dar wie ein Verfahren zum Färben oder Bedrucken von halbsynthetischem oder synthetischem Fasermaterial, insbesondere Textilmaterial, das darin besteht, eine oder mehrere Verbindungen der Formel (1) auf das genannte Material aufzubringen oder es diesem einzuverleiben. Das genannte hydrophobe Fasermaterial ist vorzugsweise textiles Polyestermaterial. Weitere Substrate, die durch das erfindungsgemässe Verfahren behandelt werden können sowie bevorzugte Verfahrensbedingungen sind vorstehend bei der näheren Erläuterung der Verwendung der erfindungsgemässen Verbindungen zu finden.

Ein weiterer Gegenstand der Erfindung ist das durch das genannte Verfahren gefärbte bzw. bedruckte hydrophobe Fasermaterial, vorzugsweise Polyester-Textilmaterial.

Die folgenden Beispiele veranschaulichen die Erfindung weiter, ohne sie darauf zu beschränken. Teile und Prozente beziehen sich, sofern nicht anderes angegeben ist, auf das Gewicht. Die Temperaturen sind in Grad Celsius angegeben.

Beispiel 1: Zu einem Gemisch, bestehend aus 14,2 g der Verbindung der Formel

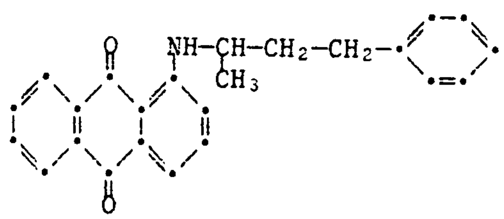

7 g Malodinitril und 250 ml Methylenchlorid lässt man bei einer Temperatur von 0-5° unter intensivem Rühren innerhalb von 30 Minuten 23 ml Titantetrachlorid zutropfen und anschliessend bei derselben Temperatur innerhalb von 30 Minuten 70 ml Pyridin. Man rührt das Gemisch anschliessend noch während 2 Stunden, wobei man die Temperatur auf 20° ansteigen lässt. Man entfernt nun die flüchtigen Anteile in einem Rotationsverdampfer im Vakuum, worauf man den Rückstand mit 200 ml 2n-HCl behandelt, abfiltriert und mit 2n-HCl nachwäscht. Man wäscht hierauf das Nutschgut mit Wasser bis zum neutralen Ablauf und anschliessend mit Methanol zur Entfernung brauner Nebenprodukte. Nach Zwischentrocknung wird das Produkt aus 10 Teilen Ethylcellosolve umkristallisiert. Man erhält den Farbstoff als dunkelviolettes Pulver in einer Ausbeute von 80 %, Fp. 196°.

Der Farbstoff entspricht der Formel

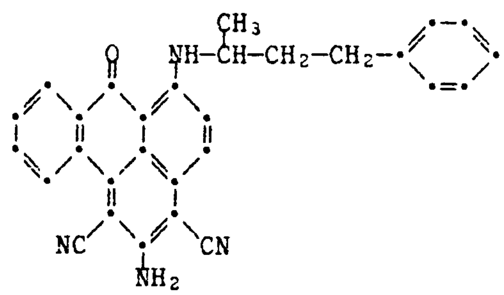

welche sich in Uebereinstimmung befindet mit der Elementaranalyse, dem Massen-, dem Protonen-Kernresonanz-Spektrum und dem $^{13}C$-Kernresonanzspektrum.

Als Dispersionsfarbstoff nach den üblichen Verfahren auf Polyester appliziert erhält man brillante blaustichigrote Färbungen mit guten Echtheitseigenschaften, insbesondere mit guten Sublimations- und Lichtechtheiten.

Beispiele 2-29: Verwendet man, bei ansonst gleichem Vorgehen, entsprechende 1-Alkylamino- bzw. Arylamino-Anthrachinone, so erhält man die in der folgenden Tabelle aufgeführten Farbstoffe, welche ähnliche Eigenschaften aufweisen und Polyester in der angegebenen Nuance färben.

Tabelle

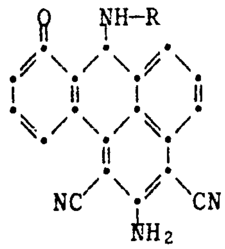

| Beispiel | R | Nuance auf Polyesterfasern |
|---|---|---|
| 2 | $-CH_3$ | blaustichig rot |
| 3 | $-C_2H_5$ | blaustichig rot |
| 4 | $-CH_2-CH_2-CH_3$ | blaustichig rot |
| 5 | 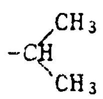 | blaustichig rot |
| 6 | $-CH_2-CH_2-CH_2-CH_3$ | blaustichig rot |
| 7 | 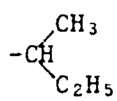 | blaustichig rot |
| 8 | 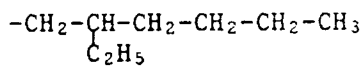 | blaustichig rot |
| 9 | 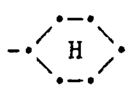 | blaustichig rot |
| 10 | 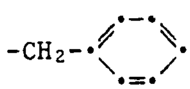 | blaustichig rot |
| 11 | 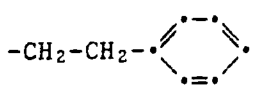 | blaustichig rot |
| 12 | 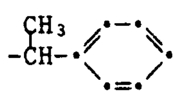 | blaustichig rot |
| 13 | 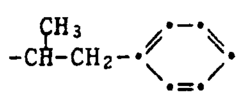 | blaustichig rot |
| 14 | 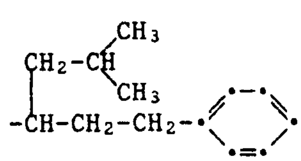 | blaustichig rot |

| Beispiel | R | Nuance auf Polyesterfasern |
|---|---|---|
| 15 | 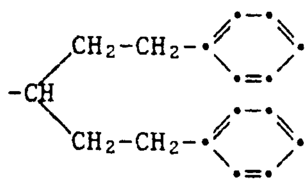 | blaustichig rot |
| 16 | 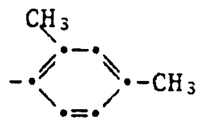 | rot-violett |
| 17 | 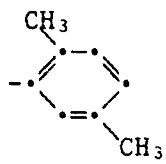 | rot-violett |
| 18 | 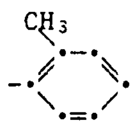 | rot-violett |
| 19 | 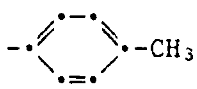 | rot-violett |
| 20 | 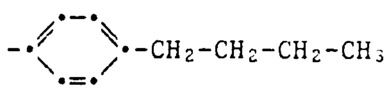 | rot-violett |
| 21 | 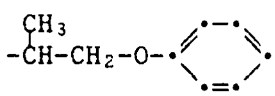 | blaustichig rot |
| 22 | 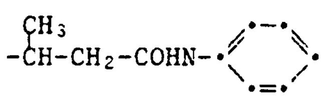 | blaustichig rot |
| 23 | 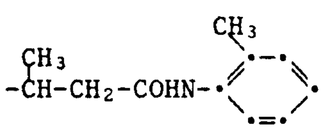 | blaustichig rot |
| 24 | 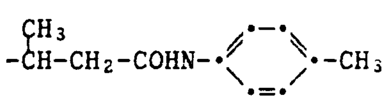 | blaustichig rot |
| 25 | 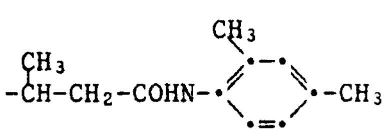 | blaustichig rot |

| Beispiel | R | Nuance auf Polyesterfasern |
|---|---|---|
| 26 | 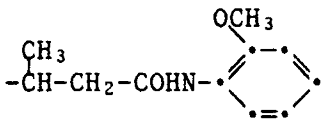 | blaustichig rot |
| 27 | 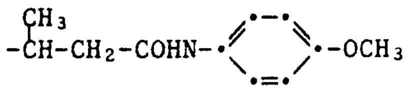 | blaustichig rot |
| 28 | 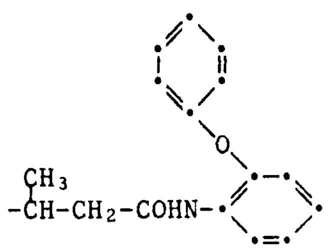 | blaustichig rot |
| 29 | 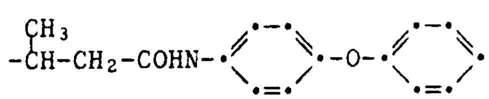 | blaustichig rot |

Beispiel 30: Man trägt 4 g des Produkts aus Beispiel 1 in 2o ml Schwefelsäure-Monohydrat ein und rührt das Gemisch während 18 Stunden bei einer Temperatur von 20-25°, worauf man auf Eis ausgiesst. Die gebildete Fällung wird abfiltriert und mit 10%-iger Kochsalzlösung gewaschen. Man schlämmt hierauf das Nutschgut mit 200 ml Wasser an, stellt den pH-Wert mit Natronlauge auf 7 und erhitzt das Gemisch auf 70°, worauf man 20 g Kochsalz einträgt. Die gebildete Fällung wird noch warm abfiltriert, mit etwas 10%-iger Kochsalzlösung gewaschen und anschliessend im Vakuum bei 60° getrocknet. Man erhält das Natriumsalz des Farbstoffs der Formel

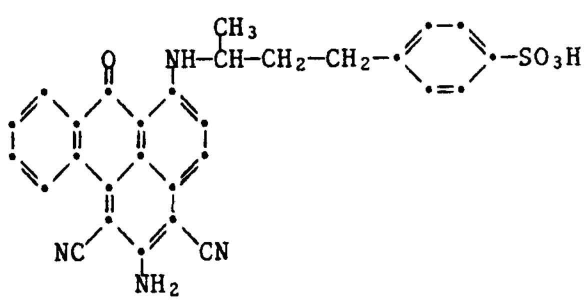

als violettes Pulver, welches sich in Wasser mit violetter Farbe löst.

Mit diesem Farbstoff lässt sich Polyamid aus schwach saurer Lösung in rot-violetten Nuancen anfärben.

Beispiel 31: Arbeitet man wie im Beispiel 1 beschrieben, setzt jedoch anstelle von 14,2 g 1-Phenylisobutylaminoanthrachinon eine äquivalente Menge 6-Chlor-1-phenylisobutylaminoanthrachinon ein, so erhält man bei ansonsten gleichem Arbeiten den Farbstoff der Formel

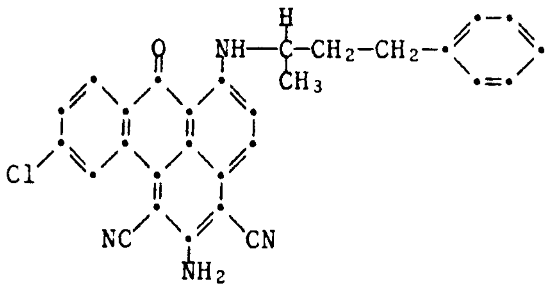

welcher Polyester in blaustichig roten Farbtönen mit guten Echtheiten färbt.

Beispiel 32: Man mischt 1 Teil des trockenen, coupagefreien Farbstoffs gemäss Beispiel 1 in einer Glasperlmühle zusammen mit 1 Teil Dinaphthylmethandisulfonat (Na-Salz) und Wasser und mahlt das Gemisch so lange, bis eine Korngrösse von etwa 2 $\mu$ oder kleiner erreicht ist. Die entstehende Paste, bestehend aus dem Farbstoff, Dispergator und Wasser, wird anschliessend mit 3 Teilen Natriumligninsulfonat versetzt. Die erhaltene Paste wird sodann der Sprühtrocknung unterworfen, wobei ein pulverförmiges Färbepräparat erhalten wird.

Dieses Färbepräparat kann zum Färben von Polyestermaterialien, z.B. nach dem HT-Verfahren, verwendet werden, wobei das Färbebad eine gute Dispersionsstabilität aufweist. Man erhält eine blaustichigrote Polyesterfärbung mit guter Lichtechtheit.

Beispiel 33: 2 Teile des gemäss Beispiel 1 erhaltenen Farbstoffs werden in 4000 Teilen Wasser dispergiert. Zu dieser Dispersion gibt man 12 Teile des Natriumsalzes von o-Phenylphenol sowie 12 Teile Diammoniumphosphat und färbt 100 Teile Garn aus Polyethylenglykolterephthalat 90 Minuten lang bei 95 bis 98° in dieser Flotte.

Die Färbung wird anschliessend gespült und mit wässriger Natronlauge und einem Dispergator nachbehandelt. Man erhält so eine brillante licht- und sublimierechte blaustichig rote Färbung.

Beispiel 34: 1 Teil des gemäss Beispiel 1 erhaltenen Farbstoffs wird mit 2 Teilen einer 50%igen wässerigen Lösung des Natriumsalzes der Dinaphthylmethandisulfonsäure nass vermahlen und getrocknet.

Dieses Farbstoffpräparat wird mit 40 Teilen einer 10%igen wässrigen Lösung des Natriumsalzes der N-Benzylheptadecyl-benzimidazoldisulfonsäure verrührt und 4 Teile einer 40%igen Essigsäurelösung zugegeben. Durch Verdünnen mit Wasser wird daraus ein Färbebad von 4000 Teilen bereitet.

In dieses Bad geht man bei 50° mit 100 Teilen eines Polyesterfaserstoffes ein, steigert die Temperatur innert einer halben Stunde auf 120 bis 130° und färbt eine Stunde in geschlossenem Gefäss bei dieser Temperatur. Anschliessend wird gut gespült. Man erhält eine brillante blaustichig rote Färbung von guter Lichtechtheit.

Beispiel 35: Polyethylenglykolterephthalatgewebe wird auf einem Foulard bei 40° mit einer Flotte folgender Zusammensetzung imprägniert:

| | |
|---|---|
| 20 Teile | des gemäss Beispiel 1 erhaltenen Farbstoffes fein dispergiert in |
| 10 Teilen | Natriumalginat, |
| 20 Teilen | Triethanolamin, |
| 20 Teilen | Octylphenolpolyglykoläther und |
| 930 Teilen | Wasser. |

Das auf ca. 100 % abgequetschte Gewebe wird bei 100° getrocknet und anschliessend während 30 Sekunden bei einer Temperatur von 210° fixiert. Die gefärbte Ware wird mit Wasser gespült, geseift und getrocknet. Man erhält eine brillante, lichtechte blaustichig rote Färbung.

**Patentansprüche**

1. Verbindungen der Formel

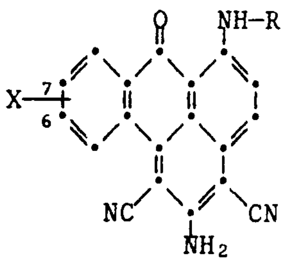

(1)

worin
R Wasserstoff, eine gegebenenfalls substituierte Alkyl- oder Arylgruppe und
X Wasserstoff oder Halogen, das sich in 6- oder 7-Stellung befindet, bedeutet.

2. Verbindungen gemäss Anspruch 1, worin R einen gegebenenfalls substituierten unverzweigten oder verzweigten Alkylrest, einen Cycloalkylrest oder einen gegebenenfalls substituierten Naphthyl-oder Phenylrest bedeutet.

3. Verbindung gemäss Anspruch 2, worin R einen offenkettigen Alkyrest mit 1 bis 8 C-Atomen bedeutet, der unsubstituiert ist oder substituiert durch OH, $C_1$-$C_4$-Alkoxy, durch OH substituiertes $C_1$-$C_4$-Alkoxy, Phenyl, Phenoxy oder Phenylaminocarbonyl, wobei die Phenylgruppe in den drei letztgenannten Resten z.B. durch $C_1$-$C_4$-Alkyl, $C_1$-$C_4$-Alkoxy oder Phenoxy substituiert sein kann.

4. Verbindungen gemäss Anspruch 2, worin R einen $C_5$-$C_8$-Cycloalkylrest bedeutet, der unsubstituiert oder durch $C_1$-$C_4$-Alkyl substituiert ist.

5. Verbindungen gemäss Anspruch 2, worin R eine Naphthyl- oder Phenylrest bedeutet, der unsubstituiert ist oder substituiert durch $C_1$-$C_4$-Alkyl, $C_1$-$C_4$-Alkoxy, Halogen, wie Fluor, Chlor oder Brom, Nitro, $C_1$-$C_4$-Alkylcarbonylamino oder $C_1$-$C_4$-Alkoxycarbonyl.

6. Verbindungen gemäss Anspruch 2, worin
R $C_1$-$C_6$-Alkyl, welches unsubstituiert ist oder durch Phenyl, Phenoxy oder Phenylaminocarbonyl substituiert ist, wobei die Phenylgruppe in den drei letztgenannten Resten durch $C_1$-$C_4$-Alkyl, $C_1$-$C_4$-Alkoxy oder Phenoxy substituiert sein kann, oder Phenyl, welches unsubstituiert ist oder durch $C_1$-$C_4$-Alkyl substituiert ist, bedeutet.

7. Verbindungen gemäss einem der Ansprüche 1-6, worin X Wasserstoff bedeutet.

8. Verfahren zur Herstellung der Verbindungen der Formel (1) gemäss Anspruch 1, dadurch gekennzeichnet, dass man ein Aminoanthrachinon der Formel

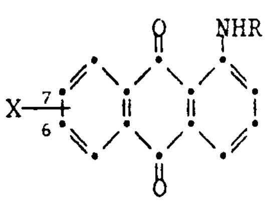

(2)

worin X und R die unter der Formel (1) angegebene Bedeutung aufweisen, in einem inerten Lösungsmittel in Gegenwart von Titantetrachlorid und einem tert.Amin mit Malodinitril umsetzt.

9. Verfahren gemäss Anspruch 8, dadurch gekennzeichnet, dass man als inertes Lösungsmittel aliphatische Kohlenwasserstoffe, chlorierte Kohlenwasserstoffe, Ether oder aromatische Verbindungen verwendet.

**10.** Verfahren gemäss Anspruch 9, dadurch gekennzeichnet, dass man einen chlorierten Kohlenwasserstoff, vor allem Methylenchlorid, verwendet.

**11.** Verfahren gemäss einem der Ansprüche 8-10, dadurch gekennzeichnet, dass man als tertiäres Amin ein aliphatisches oder aromatisches Amin verwendet.

**12.** Verfahren gemäss Anspruch 11, dadurch gekennzeichnet, dass man als tertiäres Amin Pyridin verwendet.

**13.** Verfahren gemäss einem der Ansprüche 8-12, dadurch gekennzeichnet, dass man pro Mol Anthrachinonverbindung der Formel (2) mindestens 2 Mol, vorzugsweise 2,2-3 Mol Malodinitril einsetzt.

**14.** Verfahren gemäss einem der Ansprüche 8-13, dadurch gekennzeichnet, dass man pro Mol Anthrachinonverbindung der Formel (2) 2 bis 10 Mol, insbesondere 3 bis 6 Mol Titantetrachlorid verwendet.

**15.** Verfahren gemäss einem der Ansprüche 8-14, dadurch gekennzeichnet, dass man 2 bis 6 Mol tertiäres Amin pro Mol Titantetrachlorid verwendet.

**16.** Verwendung der Verbindungen der Formel (1) gemäss Anspruch 1 zum Färben und Pigmentieren von hochmolekularen organischen Materialien.

**17.** Verwendung gemäss Anspruch 16 zum Färben oder Bedrucken von halbsynthetischen oder synthetischen Fasermaterialien, insbesondere Textilmaterialien aus linearen aromatischen Polyestern.

**18.** Verwendung gemäss Anspruch 16 zum Färben von thermoplastischen Kunststoffen in der Schmelze.

**19.** Das gemäss einem der Ansprüche 16-18 gefärbte oder bedruckte Material.

**Claims**

**1.** A compound of formula

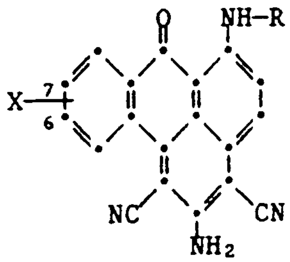

(1)

wherein
R is hydrogen or an unsubstituted or substituted alkyl or aryl group and
X is hydrogen or halogen which is in the 6- or 7-position.

**2.** A compound according to claim 1, wherein R is an unsubstituted or substituted, unbranched or branched alkyl radical, a cycloalkyl radical or an unsubstituted or substituted naphthyl or phenyl radical.

**3.** A compound according to claim 2, wherein R is an open-chain alkyl radical having 1 to 8 carbon atoms which is unsubstituted or substituted by OH, $C_1$-$C_4$alkoxy, $C_1$-$C_4$alkoxy which is substituted by OH, or by phenyl, phenoxy or phenylaminocarbonyl, it being possible for the phenyl group in the three last-mentioned radicals to be substituted by, for example, $C_1$-$C_4$alkyl, $C_1$-$C_4$alkoxy or phenoxy.

**4.** A compound according to claim 2, wherein R is a $C_5$-$C_8$cycloalkyl radical which is unsubstituted or substituted by $C_1$-$C_4$alkyl.

**5.** A compound according to claim 2, wherein R is a naphthyl or phenyl radical which is unsubstituted or substituted by $C_1$-$C_4$alkyl, $C_1$-$C_4$alkoxy, halogen such as fluorine, chlorine or bromine, nitro, $C_1$-$C_4$alkylcarbonylamino or $C_1$-$C_4$alkoxycarbonyl.

**6.** A compound according to claim 2, wherein R is $C_1$-$C_6$alkyl which is unsubstituted or substituted by phenyl, phenoxy or phenyl aminocarbonyl, it being possible for the phenyl group in the three last-mentioned radicals to be substituted by $C_1$-$C_4$alkyl, $C_1$-$C_4$alkoxy or phenoxy, or is phenyl which is unsubstituted or substituted by $C_1$-$C_4$alkyl.

**7.** A compound according to any one of claims 1 to 6, wherein X is hydrogen.

**8.** A process for the preparation of a compound of formula (1) according to claim 1, which comprises reacting an aminoanthraquinone of formula

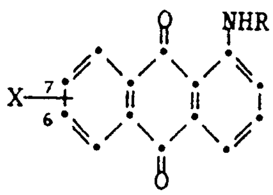

(2)

wherein X and R are as defined for formula (1), with malodinitrile in an inert solvent and in the presence of titanium tetrachloride and a tertiary amine.

**9.** A process according to claim 8, wherein the inert solvent used is an aliphatic hydrocarbon, a chlorinated hydrocarbon, an ether or an aromatic compound.

**10.** A process according to claim 9, wherein a chlorinated hydrocarbon, in particular methylene chloride, is used.

**11.** A process according to any one of claims 8 to 10, wherein the tertiary amine used is an aliphatic or aromatic amine.

**12.** A process according to claim 11, wherein the tertiary amine used is pyridine.

**13.** A process according to any one of claims 8 to 12, wherein 2 moles, preferably 2.2 to 3 moles, of malodinitrile are used per mole of anthraquinone compound of formula (2).

**14.** A process according to any one of claims 8 to 13, wherein 2 to 10 moles, in particular 3 to 6 moles, of titanium tetrachloride are used per mole of anthraquinone compound of formula (2).

**15.** A process according to any one of claims 8 to 14, wherein 2 to 6 moles of tertiary amine are used per mole of titanium tetrachloride.

**16.** The use of a compound of formula (1) according to claim 1 for dyeing and pigmenting high molecular weight organic materials.

**17.** The use according to claim 16 for dyeing or printing semi-synthetic or synthetic fibre materials, in particular textile materials made from linear aromatic polyesters.

**18.** The use according to claim 16 for colouring thermoplastics in the melt.

**19.** A material which is dyed or coloured or printed according to any one of claims 16 to 18.

**Revendications**

**1.** Composés de formule :

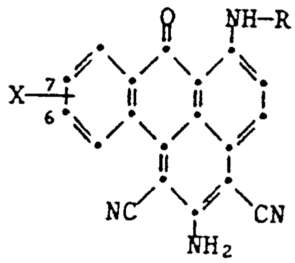

(1)

dans laquelle R représente un atome d'hydrogène ou un groupe alkyle ou aryle éventuellement substitué, et X représente un atome d'hydrogène ou d'halogène, qui se trouve en position 6 ou 7.

**2.** Composés conformes à la revendication 1, dans lesquels R représente un groupe alkyle ramifié ou non, éventuellement substitué, un groupe cycloalkyle ou un groupe phényle ou naphtyle éventuellement substitué.

**3.** Composé conforme à la revendication 2, dans lequel R représente un groupe alkyle à chaîne ouverte comportant de 1 à 8 atomes de carbone, non substitué ou substitué par des groupes -OH, alcoxy en $C_1$-$C_4$, alcoxy en $C_1$-$C_4$ hydroxysubstitué, phényle, phénoxy ou phénylaminocarbonyle, les groupes phényle, dans les trois derniers groupes cités, pouvant être substitués par exemple par des groupes alkyle en $C_1$-$C_4$, alcoxy en $C_1$-$C_4$ ou phénoxy.

**4.** Composés conformes à la revendication 2, dans lesquels R représente un groupe cycloalkyle en $C_5$-$C_8$, substitué ou non par un groupe alkyle en $C_1$-$C_4$.

**5.** Composés conformes à la revendication 2, dans lesquels R représente un groupe phényle ou naphtyle, substitué ou non par des groupes alkyle en $C_1$-$C_4$, alcoxy en $C_1$-$C_4$, nitro, (alkyl en $C_1$-$C_4$)-carbonylamino ou (alcoxy en $C_1$-$C_4$)-carbonyle, ou par des atomes d'halogène, comme des atomes de fluor, de chlore ou de brome.

**6.** Composés conformes à la revendication 2, dans lesquels R représente un groupe alkyle en $C_1$-$C_6$, substitué ou non par un groupe phényle, phénoxy ou phénylaminocarbonyle, les groupes phényle des trois groupes cités en dernier pouvant être substitués par des groupes alkyle en $C_1$-$C_4$, alcoxy en $C_1$-$C_4$ ou phénoxy, ou bien encore un groupe phényle, substitué ou non par un ou plusieurs groupes alkyle en $C_1$-$C_4$.

**7.** Composés conformes à l'une des revendications 1 à 6, dans lesquels X représente un atome d'hydrogène.

**8.** Procédé de préparation des composés de formule (1) conformes à la revendication 1, caractérisé en ce que l'on fait réagir une aminoanthraquinone de formule :

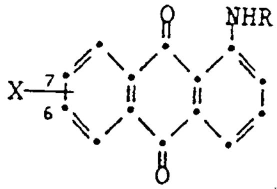

(2)

dans laquelle X et R ont les significations indiquées à propos de la formule (1), avec du malodinitrile, dans un solvant inerte, et en présence de tétrachlorure de titane et d'une amine tertiaire.

9. Procédé conforme à la revendication 8, caractérisé en ce que l'on utilise, en tant que solvant inerte, des hydrocarbures aliphatiques, des hydrocarbures chlorés, des éthers ou des composés aromatiques.

10. Procédé conforme à la revendication 9, caractérisé en ce que l'on utilise un hydrocarbure chloré, et surtout du chlorure de méthylène.

11. Procédé conforme à l'une des revendications 8 à 10, caractérisé en ce que l'on utilise, comme amine tertiaire, une amine aliphatique ou aromatique.

12. Procédé conforme à la revendication 11, caractérisé en ce que l'on utilise de la pyridine comme amine tertiaire.

13. Procédé conforme à l'une des revendications 8 à 12, caractérisé en ce que l'on utilise, par mode d'anthraquinone de formule (2), au moins 2 moles et de préférence de 2,2 à 3 modes de malodinitrile.

14. Procédé conforme à l'une des revendications 8 à 13, caractérisé en ce que l'on utilise, par mole d'anthraquinone de formule (2), de 2 à 10 moles et en particulier de 3 à 6 modes de tétrachlorure de titane.

15. Procédé conforme à l'une des revendications 8 à 14, caractérisé en ce que l'on utilise de 2 à 6 modes d'amine tertiaire par mode de tétrachlorure de titane.

16. Utilisation des composés de formule (1) conformes à la revendication 1 pour la teinture et la pigmentation de matériaux organiques à masse moléculaire élevée.

17. Utilisation conforme à la revendication 16, pour la teinture ou l'impression de matières en fibres semi-synthétiques ou synthétiques, et en particulier de matières textiles en polyester aromatique linéaire.

18. Utilisation conforme à la revendication 16, pour la teinture de matières thermoplastiques dans la masse fondue.

19. Matière teinte ou imprimée conformément à l'une des revendications 16 à 18.